Europäisches Patentamt

European Patent Office

Office européen des brevets

(11) Publication number: **0 327 390**
A2

# (12) EUROPEAN PATENT APPLICATION

(21) Application number: **89301086.8**

(22) Date of filing: **03.02.89**

(51) Int. Cl.⁴: **C 12 N 15/00**
**C 12 Q 1/68**

(30) Priority: **05.02.88 GB 8802667**

(43) Date of publication of application:
**09.08.89 Bulletin 89/32**

(84) Designated Contracting States:
**AT BE CH DE FR GB IT LI NL SE**

(71) Applicant: **ISIS INNOVATION LIMITED**
**2 South Parks Road**
**Oxford OX1 3UB (GB)**

(72) Inventor: **Wakefield, Ann Elizabeth**
**Park View Woodstock Road**
**Charlbury Oxford OX7 3ET (GB)**

**Hopkin, Julian Meurglyn**
**20B, Plantation Road**
**Oxford OX2 6JD (GB)**

**Moxon, Edward Richard**
**17 Moreton Road**
**Oxford OX2 7AX (GB)**

(74) Representative: **Pennant, Pyers et al**
**Stevens, Hewlett & Perkins 5 Quality Court Chancery Lane**
**London, WC2A 1HZ (GB)**

(54) **Probe for pneumocystis carinii.**

㉗ The invention provides DNA probes which hybridize to the DNA of Pneumocystis carinii but not to mammalian DNA, and which form the basis of an in vitro diagnostic test for viable P.carinii organisms in respiratory secretion or even sputum. The probes are made by recombinant DNA technology from infected rat lung tissue. Preferred in the plasmid designated pAZ112, derived from the vector pUC13, having a size of approximately 9.8 kbp and which has been partly sequenced.

EP 0 327 390 A2

**Description**

## PROBE FOR PNEUMOCYSTIS CARINII

Pneumocystis carinii infects the lungs of many mammals including rat and man [1,2]. The organism has been tentatively classified as a protozoan although recent data from ribosomal RNA sequence analysis suggests it may be a fungus[3]. Two phases are recognised in its life cycle, the trophozoite (1-5 microns in diameter) that adheres to the type I alveolar pneumocyte and the cyst (4-7 microns) characterised by a thick wall that surrounds 4 to 8 sporozoites with single nuclei disposed in a ring manner[4]. Significant disease follows infection typically if the host has deficient cell mediated immunity[5]. The alveolar spaces of the lung can then become progressively filled with exudates containing P.carinii to produce respiratory failure[6]. Currently, reliable diagnosis is based on microscopy of material obtained by potentially hazardous invasive lung sampling techniques[7,8] and prevention and treatment on the use of chemotherapeutic agents whose efficacy is often restricted by toxic reactions especially in the acquired immunodeficiency syndrome[9]. There is a need for a more basic understanding of the organism's biology for devising new strategies to diagnose, treat and in the long term prevent P.carinii pneumonia. Such progress has been impeded by failure to successfully propagate the organism in vitro[10].

This invention takes a step towards fulfilling that need by providing a DNA probe produced by recombinant DNA technology which hybridises specifically to the DNA of Pneumocystis carinii but not to mammalian DNA nor any other lung pathogen. As described hereafter, three specific probes of this kind are based on recombinant plasmids designated pAZ101, pAZ102 and pAZ112. The invention also encompasses micro-organisms capable of producing the said recombinant plasmids.

In another aspect, the invention provides a method of making a DNA probe for Pneumocystis carinii, which method comprises the steps of - extracting P.carinii from infected lung tissue of a mammal,
- recovering DNA from the P.carinii, inserting the DNA by ligation into a vector and using the resulting recombinant vectors to transform micro-organisms,
- screening the transformed micro-organisms for hybridization to DNA of the mammal, selecting ones which do not hybridise, and extracting recombinant vectors from the selected micro-organisms,
- screening the extracted recombinant vectors by in situ hybridization with lung tissue of a mammal infected with P.carinii, and selecting recombinant vectors that do hybridize, and
- culturing the micro-organisms that produce the selected recombinant vectors.

The method preferably involves the following steps:
- The P.carinii micro-organisms are extracted from the lungs of infected rats, and separated from rat lung tissue by filtration, the micro-organisms being generally smaller than the particles of lung tissue.
- Genomic DNA is recovered from the micro-organisms and ligated into a vector, preferably a plasmid, or alternatively a phage such as phage lambda. The resulting recombinant vectors are used to transform micro-organisms, preferably of the species E.coli.
- Transformants containing inserted DNA are subjected to negative screening, by colony hybridization with labelled rat DNA.
- Recombinant plasmids or phages are extracted from colonies which do not hybridise to rat DNA, labelled, and screened for hybridization with rat DNA, human DNA and with DNA from P.carinii.
- Those recombinant plasmids or phages which hybridise with the latter but not the former are selected and screened by in situ hybridization with P.carinii infected rat lung tissue.
- If desired, the insert DNA may be excised from the recombinant plasmids or phages and used alone to achieve a higher level of probe specificity.

In yet another aspect, the invention provides an in vitro method of diagnosing Pneumocystis carinii infection, which method comprises contacting DNA, from respiratory secretion of a patient suspected of being infected, under hybridising conditions with a probe as described. If desired, the DNA in the sample can be extracted and subjected to gene amplification in order to increase its concentration prior to the hybridisation step. This technique is expected to be sensitive enough to permit the use of sputum as the respiratory secretion. The method permits a reliable diagnosis to be achieved in a few hours, without the need for invasive lung sampling.

Reference is directed to the accompanying drawings in which:

Figure 1 is a preliminary map of the recombinant plasmid pAZ112 showing the EcoRI and Hind III restriction sites;

Figure 2 is an incomplete sequence of an EcoRI fragment of the insert, whose position is shown in Figure 1; and

Figure 3 is a sequence of pAZ112 insert DNA from the cloning site to the EcoRI 0.45 fragment, whose position is also shown in Figure 1.

Probes based on the recombinant plasmid pAZ112 are particularly preferred. The plasmid has a length of approximately 9.8 kbp and includes sites for restriction endonucleases as set out in Table 2 below. As a probe, pAZ112 has the following advantages: it appears to hybridize to a region of DNA in the P.carinii genome which is present in many copies, thus increasing the sensitivity of the assay; it appears to hybridize only with viable P.carinii organisims, and not with other organisms commonly found in respiratory fluid of infected individuals.

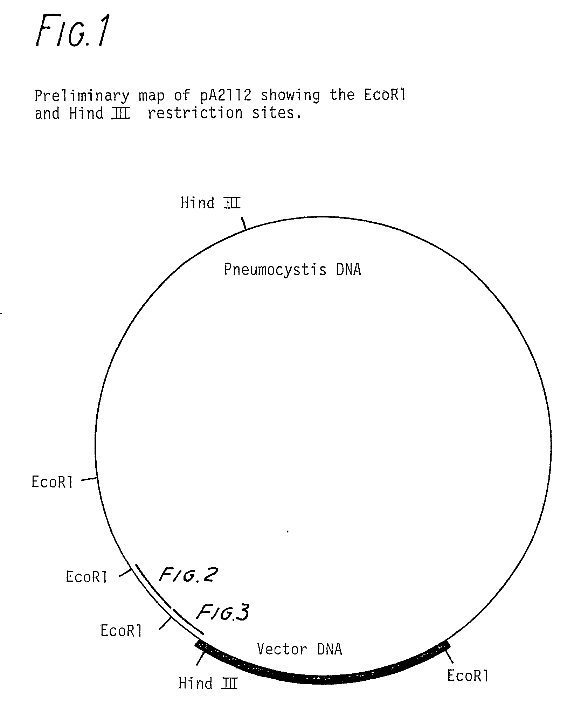

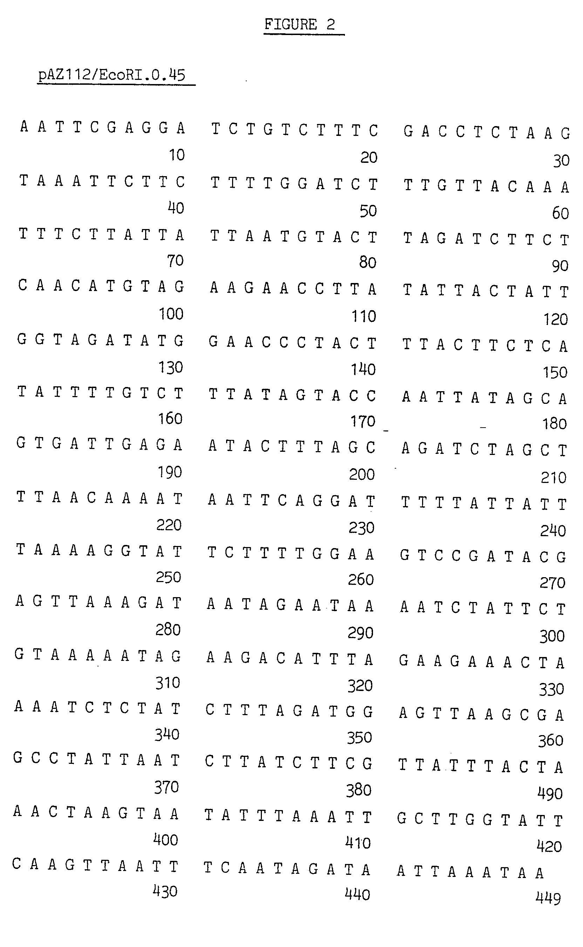

Preferably, a probe according to the invention comprises the whole or a part at least 15 nucleotides in length of the cloned insert of pAZ112. The probe may be one which includes a sequence of at least 15 nucleotides which has at least 70% homology with the sequence shown in Figure 2 or with the complimentary sequence.

As is generally the case, probes of this invention need to be capable of being detected. If the probe is immunogenic, detection may be by means of a labelled antibody. More usually, the probe will include at least one marker atom or group. Marker groups are well known in the art and may include, for example, radio-active atoms such as $^{32}$P, biotin groups or enzymes or other components of chemiluminscent or enzyme-based detection systems.

The rat model[11] of P.carinii infection was chosen as a source of parasites. Spraque-Dawley rats (170-200g) were maintained on normal diet and provided with water containing dexamethasone (2 mg/l) to induce a state of immunosuppression and tetracycline (500 mg/l) to prevent early deaths from bacterial infection. At 8 to 12 weeks the rats were sacrificed and the presence of P.carinii infection confirmed by Giemsa and

Toluidine blue staining of impression smears from the lungs[12,13]. The organisms were extracted from the lungs by extensive lavage with phosphate buffered saline and purified by differential filtration. Extracellular DNA was digested with DNase, the cysts disrupted by treatment with protease XXV and the P.carinii enriched DNA extracted.

This DNA was then used in a cloning procedure in which the vector, plasmid pUC13 (2.7 kb)[14], was linearized with Bam H1 and treated with alkaline phosphatase. The P.carinii enriched DNA was partially digested with Sau 3AI, then ligated with the vector and the resulting recombinant plasmids used to transform E.coli JM109. Ampicillin-resistant transformants were probed by the technique of colony hybridization[15] using $^{32}$P-labelled rat DNA extracted from whole blood. Forty six colonies which did not hybridise with rat DNA, were identified. The plasmids from many of these colonies were extracted, linearized and re-examined by probing with both $^{32}$P-labelled genomic rat DNA and the P.carinii enriched DNA using the technique of Southern hybridization. A number of the plasmids which hybridised with the P.carinii enriched DNA but not with rat DNA were selected for further study. These plasmids were label led with $^{32}$P by nick-translation and used to probe rat, human and P.carinii enriched DNA. Hybridization specificity of the recombinant plasmids pAZ101, pAZ102 and pAZ112 to EcoRI-digested rat DNA; Sau 3AI-digested rat DNA; EcoRI-digested human DNA; Sau 3AI-digested human DNA; EcoRI-digested P.carinii enriched DNA; Sau 3AI-digested P.carinii enriched DNA; was determined. Three recombinant plasmids, pAZ101, pAZ102 and pAZ112, hybridised to the P.carinii enriched DNA but not to rat or human DNA. In contrast, the vector pUC13, showed no hybridistion with any of the DNA samples. The approximate size of the recombinant plasmid pAZ101 was 5.4 kb, pAZ102 was 3.3 kb, and pAZ112 was 9.8 kb.

E.coli JM109 (pAZ101) and E.coli JM109 (pAZ102) and Ecoli JM109 (pAZ112) were deposited with the National Collections of Industrial and Marine Bacteria Ltd. as follows:

| Date | Strain | NCIB Number |
|------|--------|-------------|
| 28 January 1988 | JM109 (pAZ101) | 12634 |
| 28 January 1988 | JM109 (pAZ102) | 12635 |
| 16 January 1989 | JM109 (pAZ112) | 40101 |

Other recombinant plasmids have been made by the same method and shown to have generally the same properties as PAZ101, 102 and 112. These are:

| Recombinant Plasmid | Approx. size, bp |
|---|---|
| pAZ103 | 6500 |
| pAZ104 | 3200 |
| pAZ105 | 7100 |
| pAZ106 | 3100 |
| pAZ107 | 7500 |
| pAZ108 | 4200 |
| pAZ109 | 4560 |
| pAZ110 | 4370 |
| pAZ111 | 2860 |
| pAZ113 | 2840 |
| pAZ114 | 2750 |
| pAZ115 | 3300 |
| pAZ116 | 2870 |
| pAZ117 | 2870 |
| pAZ118 | 2900 |
| pAZ119 | 8500 |
| pAZ120 | 5300 |

The specificity of pAZ101, pAZ102 and pAZ112 for P.carinii was investigated using the technique of non-isotopic in situ hybridization[16]. The vector plasmid pUC13 and a human papilloma virus probe (HPV6) were used for comparison. Sections of uninfected rat and human lungs, histologically confirmed P.carinii infected lungs and wart virus infected cervical biopsies were prepared from formalin fixed, paraffin embedded specimens. The probes were biotinylated and visualisation was carried out using avidin conjugated to alkaline phosphatase with nitro blue tetrazolium and 5-bromo-4-chloro-3-indolyl phosphate as substrate. Pyronin was used as a counterstain.

The in situ hybridisation studies (Table 1) showed that hybridisation occured between pAZ101, pAZ102 or pAZ112 and DNA in exudates in the alveolar spaces of infected rat and human lungs but did not occur with normal lungs. The vector pUC$_{13}$ and the papilloma virus probe, HPV6, did not hybridise with either. The pattern of binding produced by pAZ101, 102 and 112 was characteristic of the distribution of P.carinii infection, being confined to alveolar space exudates and sparing the interstitium of the lung and bronchi.

Further studies were performed on samples of lung tissue from rats, (infected and uninfected) and humans (infected and uninfected), to test the specificity of pAZ101 and 102 by comparing these results to those obtained using vector probes (pUC13 and pBR322) and probes derived from human papilloma virus (HPV6 and HP16)[16], from Herpes simplex and cytomegalovirus under conditions of high and low stringency. The results consistently showed that only pAZ101, pAZ102 and pAZ112 hybridised to lung infected with P.carinii.

The dominant signals seem under high power were 4-6 micron ring structures many visibly comprised of 2 to 8 one micron beaded nuclei, typical of the cyst phase of P.carinii with its sporozoites. Punctate hybridization signals seen in the exudates were considered to represent hybridization to nuclei of the trophozoite phase of the organism. These results indicate that clones pAZ101, pAZ102 and pAZ112 do not hybridise with mammalian DNA but produce patterns of specific binding to P.carinii that conform to the typical distribution of morphology of the organism in infected lung of both rats and humans. The findings suggest that the cloned DNA sequences are derived from P.carinii and since the plasmids pAZ101, 102 and 112 reacted with P.carinii in both humans and rat lungs and with similar intensity under the same conditions of stringency, the sequences of DNA present in these clones are presumed to be common to the parasites infecting these different hosts.

Table 1

In situ hybridization of probe DNA to infected and unifected tissue.

| | Probe | | | | |
| | pAZ101 | pAZ102 | pAZ112 | pUC13 | HPV6 |
|---|---|---|---|---|---|
| uninfected rat lung | - | - | - | - | - |
| P.carinii infected rat lung | + | + | + | - | - |
| uninfected human lung | - | - | - | - | - |
| P.carinii infected human lung | + | + | + | - | - |
| wart virus infected human cervix | - | - | | - | + |

pAZ112

The recombinant plasmid pAZ112 has been shown to have a size of approximately 9.8 Kbp, and to be acted on by restriction endonucleases in the manner set out in the following Table 2.

Table 2

| Restriction endonuclease | Recognition sequence | No. of sites in pAZ112 at least | No. of sites in vector (pUC13) | No. of sites in P.c DNA at least |
|---|---|---|---|---|
| Hae III | GG'CC | > 8 | 11 (587bp-llbp) | 2 |
| Apa I | GGGCC'C | 0 | 0 | 0 |
| Sma I | CCC'GGG | 1 | 1 | 0 |
| Dra I | TTT'AAA | ≧ 11 | 3 (1975bp 692bp 19 bp) | 9 |
| Hind III | A'AGCTT | 2 | 1 | 1 |
| EcoRI | G'AATTC | 4 | 1 | 3 |
| Xba I | T'CTAGA | 4 | 1 | 3 |
| Pst I | CTGCA'G | 1 | 1 | 0 |
| Pvu II | CAG'CTC | 2 | 2 | 0 |
| Sal I | G'TCGAC | 1 | 1 | 0 |

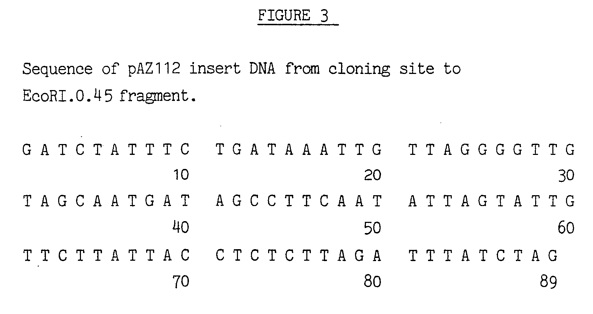

Sequencing has been performed on part of the insert of plasmid pAZ112. Figure 2 gives the incomplete nucleotide sequence of an EcoR1 fragment about 0.45 Kbp long. Figure 3 gives the sequence of an adjacent fragment extending to the cloning site.

The cloned DNA in pAZ112 has a high content of the bases adenine and thymine as determined by digestion with restriction endonucleases with different recognition sequences. For example, the EcoR1 fragment shown in Figure 2 contains 74% A and T, and only 26% of C and G.

When total genomic DNA of P.carinii is digested with the restriction endonuclease HaeIII, which has a tetranucleotide recognition sequence of GGCC, and the resulting fragments subjected to electrophoresis, a band is seen which consists of a DNA sequence approximately 9 Kbp long which is present in many copies within the genome. This band is absent in HaeIII digests of genomic rat DNA. The recombinant plasmid pAZ112 hybridizes with this 9 Kb band, whereas the vector plasmid pUC13 shows no binding.

This proves that part or all of the cloned insert of pAZ112 hybridizes with a region of DNA in the genome of P.carinii which is present in many copies. This is significant, because it means that probes based on pAZ112, and any other probes that bind to multiple copy regions, can be used to improve the sensitivity of an assay for P.carinii.

As a probe for P.carinii, pAZ112 has been shown to be rather specific. As indicated above, it does not hybridize to mammalian DNA. It has also been shown by Southern blot techniques not to hybridize to other lung pathogens, specifically Candida albicans, a non-albicans species of Candida and Cryptococcus neoformans.

In a small scale blind clinical trial, pAZ112 was used to detect the presence of P.carinii in respiratory tract samples by in situ hybridisation. Preliminary results show that this technique was more sensitive than conventional staining. It detected organisms in samples from patients who were clinically diagnosed as having pneumocystis pneumonia and who responded to treatment for the disease, but in whose samples conventional staining failed to show the presence of P.carinii. Moreover, the test appears to only detect viable organisms.

Methods

Rat DNA was extracted from blood, obtained by cardiac puncture of four normal adult rats. Human DNA was obtained form the venous blood of 5 volunteers. P.carinii was prepared by differential filtration of the lavage fluid of lungs of rats with proven P.carinii infection. Coarse (sintered glass, 40 - 90 microns) and fine (Nuclepore 8, 5, 3 microns) filters were used.

This yielded trophoziotes and cysts in a ratio of approximately 50:1; contamination with intact rat cells and nuclei was <1%. The filtrate was digested with DNAse (Sigma) at a final concentration of 1 μg/ml in the presence of 5 mM Mg $Cl_2$ at 37°C for 1 h. The P.carinii enriched preparation was washed with buffer (0.01 M Tris, pH 7.8, 0.01 M EDTA, pH 8.0), resuspended to a final concentration of 0.2% SDS and digested with protease XXV (Sigma, self-digested, final concentration of 1 mg/ml) at 37°C for 16h. The DNA was extracted with phenol and chloroform followed by ethanol precipitation.

Following restriction endonuclease digestion, DNA samples were electrophoresed on 0.8% agarose gels and transferred to nitrocellulose filters. Vector and recombinant plasmid probes were labelled with [32P] by nick-translation. Hybridization was performed in the presence of 50% formamide, 5 x Denhardt's solution, 3 x SSC, 0.1% SDS and 100 ug/ml E.coli tRNA at 42°C for 16 h. The filters were washed in 2 x SSC, 0.1% SDS at 22°C for 5 x 15 min, then 0.1 x SSC, 0.1% SDS at 60°C for 2 x 15 min and autoradiographed.

Sections were cut form formalin fixed, paraffin embedded tissue specimens. The DNA probes were biotinylated[17] by nick-translation (Amersham) with biotin-dUTP (Bethesda Research Laboratories). In situ hybridization was performed according to Burns et al[17] with the following modifications. Pepsin/HCl treatment of sections was performed at 37°C for 15 min followed by 5 washes in PBS and air drying. After hybridization the slides were washed in 4 x SSC at 22°C for 5 x 5 min, 2 x SSC at 22°C for 5 min, 0.1 x SSC at 60°C for 5 min (high stringency) or in 4 x SSC at 22°C for 5 x 5 min (low stringency) followed by a 10 min wash in blocking buffer (0.05M Tris, pH 7.2, 0.1 M NaCl, 0.001 M MgCl2, 0.05% Triton X-100, 2% BSA). The hybridised biotinylated probes were visualised using avidin (modified) - alkaline phosphatase conjugate (Dako) diluted 1 in 25 in blocking buffer, and nitro blue tetrazolium with 5-bromo-4-chloro-3-indolyl phosphate (NBT-BCIP) as substrate. The sections were counterstained in 50% pyronin for 5 sec and mounted in glycerine jelly.

Sensitivity tests with sputum were performed generally as above. But the DNA was treated for 15 minutes at 37°C with proteinase K at 500 mg/ml (cyst) or 10 μg/ml (trophozoite). And the DNA was denatured at 75°C.

References

1. Frenkel, J.K. & Havenhill, M.A. Lab. Invest. 12, 1204-1220 (1963).

2. Meuwissen, J.H.E.Th., Tauber, I., Leeuwenberg, A.D.E.M., Beckers, P.J.A. & Sieben, M. J. infect. Dis. 136, 43-49 (1977).

3. Edmans, J.6. Kovacs, J.A. Masur, H., Santi, D.V., Elwood, H.J. & Sogin, M.L. Nature 334, 519-522 (1988)

4. Campbell, W.G. Arch. Path. 93, 312-324 (1972).

5. Walzer, P.D., Perl, D.P. Krogstad, D.J., Rawson, P.G. & Schultz, M.G. in Symposium on Pneumocystis carinni infection (eds. robbins, J.B., DeVita, V.T. & Dutz, W.) 55-63 (U.S. Dept. of Health, Bethesda, 1976).

6. Engleberg, L.A., Lerner, C.W. & Tapper, M.L. Am. Rev. Respir. Dis. 130, 689-694 (1984).

7. Hopkin, J.M., Turney, J.H., Young, J.A., Adu, D. & Michael, J. Lancet ii, 299-301 (1983).

8. Craddock, C., Pasvol, G., Bull, R., Protheroe, A. & Hopkins, J.M. Lancet ii, 16-18 (1987).

9. Gordin, F.M., Simon, G.L. Wofsy, C.B. & Mills, J. Ann. intern. Med. 100, 495-499 (1984).

10. Smith, J.W. & Bartlett, M.S. in Pneumocystis carinii pneumonia (ed. Young, L.S.) 107-137 (Dekker, New York, 1984).

11. Gigliotti, F., Stokes, D.C., Cheatham, A.B., Davis, D.S. & Hughes, W.T. J. infect. Dis. 154, 315-322 (1986).

12. Masur, H. & Jones, T.C. J. exp. Med. 147, 157-170 (1978).

13. Chalvardjian, A.M. & Grawe, L.A. J. clin. Path. 16, 383-384 (1963).

14. Messing, J. Meth. Enzym. 101, 20-78 (1983).

15. Grunstein, M. & Hogness, D.S. Proc. patn. Acad. Sci. U.S.A. 72, 3961-3965 (1975).

16. Burns, J., Graham, A.K., Frank, C., Fleming, K.A., Evans, M.F. & McGee, J.O. 'D. J. clin. Path. 40, 858-864 (1987).

17. Ferguson, D.J.P., Burns, J., Harrison, D., Jonasson, J. & McGee, J.O. 'D. Histochem. J. 18, 266-270 (1986).

**Claims**

1. A DNA probe produced by recombinant DNA technology which hybridizes to the DNA of Pneumocystis carinii but not to mammalian DNA.

2. A probe as claimed in claim 1 which comprises part or all of the insert of the plasmid designated pAZ101.

3. A probe as claimed in claim 1 which comprises part or all of the insert of the plasmid designated pAZ102.

4. A probe as claimed in claim 1 which comprises part or all of the insert of the plasmid designated pAZ112.

5. A probe as claimed in claim 4, wherein the insert has a length of approximately 7.1 kbp and includes the following sites for the following restriction endonucleases:

| Restriction endonuclease | Minimum number of sites in insert |
|---|---|
| Hae III | 2 |
| Dra I | 9 |
| Hind III | 1 |
| EcoR I | 3 |
| Xba I | 3 |
| Apal, Smal, Pstl, Pvull, Sall | 0 |

6. A probe as claimed in claim 4 or claim 5, wherein the insert includes a DNA sequence which is substantially the sequence shown in Figure 2.

7. A DNA probe which hybridizes to the DNA of P.carinii but not to mammalian DNA, which probe includes a sequence of at least 15 nucleotides which has at least 70% homology with the sequence shown in Figure 2 or with the complementary sequence.

8. A probe as claimed in any one of claims 1 to 7, which hybridizes to a DNA sequence that is present in more than one copy in the genome of P.carinii.

9. A probe as claimed in any one of claims 1 to 8, wheron at least one marker atom or group is present.

10. A micro-organism which is capable of producing the DNA of any one of claims 1 to 8.

11. A method of making a DNA probe for Pneumocystis carinii, which method comprises the steps of-
extracting P.carinii from infected lung tissue of a mammal,
- recovering DNA from the P.carinii, inserting the DNA by ligation into a vector and using the resulting recombinant vectors to transform microorganisms,
- screening the transformed micro-organisms for hybridization to DNA of the mammal, selecting ones which do not hybridise, and extracting recombinant vectors from the selected micro-organisms,
- screening the extracted recombinant vectors by in situ hybridization with lung tissue of a mammal infected with P.carinii, and selecting recombinant vectors that do hybridize, to the P.carinii, and
- culturing the micro-organisms that produce the selected recombinant vectors.

12. A method as claimed in claim 11, wherein the mammal is a rat, and the P.carinii is separated from rat lung tissue by filtration.

13. A method as claimed in claim 11 or claim 12, wherein the vector is a plasmid and the micro-organisms are of the species E. coli.

14. A method as claimed in any one of claims 11 to 13, wherein the in situ hybridization step is performed using an enzyme label.

15. An in vitro method of diagnosing Pneumocystis carinii infection, which method comprises contacting DNA, from respiratory secretion of a patient suspected of being infected, under hybridizing conditions with the probe claimed in any one of claims 1 to 9 or the probe made by the method of any one of claims 11 to 14.

16. An in vitro method as claimed in claim 15, wherein the respiratory secretion is sputum.

# *FIG.1*

Preliminary map of pA2112 showing the EcoR1
and Hind Ⅲ restriction sites.

Hind Ⅲ

Pneumocystis DNA

EcoR1

EcoR1 — *FIG.2*

*FIG.3*

EcoR1

Vector DNA

Hind Ⅲ

EcoR1

FIGURE 2

pAZ112/EcoRI.0.45

AATTCGAGGA    TCTGTCTTTC    GACCTCTAAG

TAAATTCTTC    TTTTGGATCT    TTGTTACAAA

TTTCTTATTA    TTAATGTACT    TAGATCTTCT

CAACATGTAG    AAGAACCTTA    TATTACTATT

GGTAGATATG    GAACCCTACT    TTACTTCTCA

TATTTTGTCT    TTATAGTACC    AATTATAGCA

GTGATTGAGA    ATACTTTAGC    AGATCTAGCT

TTAACAAAAT    AATTCAGGAT    TTTTATTATT

TAAAAGGTAT    TCTTTTGGAA    GTCCGATACG

AGTTAAAGAT    AATAGAATAA    AATCTATTCT

GTAAAAATAG    AAGACATTTA    GAAGAAACTA

AAATCTCTAT    CTTTAGATGG    AGTTAAGCGA

GCCTATTAAT    CTTATCTTCG    TTATTTACTA

AACTAAGTAA    TATTTAAATT    GCTTGGTATT

CAAGTTAATT    TCAATAGATA    ATTAAATAA

FIGURE 3

Sequence of pAZ112 insert DNA from cloning site to
EcoRI.0.45 fragment.

```
G A T C T A T T T C   T G A T A A A T T G   T T A G G G G T T G
                 10                   20                   30
T A G C A A T G A T   A G C C T T C A A T   A T T A G T A T T G
                 40                   50                   60
T T C T T A T T A C   C T C T C T T A G A   T T T A T C T A G
                 70                   80                 89
```